# EUROPEAN PATENT APPLICATION

(11) **EP 1 205 471 A1**
(43) Date of publication of application: **15.05.2002**
(21) Application number: 01103018.6
(22) Date of filing: 08.02.2001
(51) Int. Cl.: C07C 391/00, A23L 1/305

(54) **A method of using synthetic L-SE-Methylselenocysteine as a nutriceutical and a method of its synthesis**

(30) Priority: 02.10.2000 US 677563
(71) Applicant: PharmaSe, Incorporated, Lubbock, Texas 79413 (US)
(72) Inventor: Spallholz, Julian, E, Texas 79423 (US); Reid, Ted W, Texas 79424 (US); Walkup, Robert D, Texas 79363 (US)
(74) Representative: Frankland, Nigel Howard

(57) **Abstract**

A synthesis of and use of L-Se-methylselenocysteine as a nutriceutical is described, based upon the knowledge that L-Se-methylselenocysteine is less toxic than L-selenomethionine towards normal cells. The synthesis proceeds by mixing N-(tert-butoxycarbonyl)-L-serine with a dialkyl diazodicarboxylate and at least one of a trialkylphosphine, triarylphosphine and phosphite to form a first mixture that includes N-(tert-butoxycarbonyl)-L-serine β-lactone. Methyl selenol or its salt is mixed with the N-(tert-butoxycarbonyl)-L-serine β-lactone to form a second mixture that includes N-(tert-butoxycarbonyl)-Se-methylselenocysteine. The text butoxycarbonyl group is removed from the N-(tert-butoxycarbonyl)-Se-methylselenocysteine to form L-Se-methylselenocysteine. This synthesis significantly improves the manufacturability, manufacturing efficiency and utility of this naturally occurring rare form of organic-selenium. L-Se-methylselenocysteine formed, for example, in this manner may be used as a nutriceutical for supplementation into the diets of humans or animals for various beneficial purposes, such as, for example, to prevent or reduce the risk of developing cancer.

## Description

### BACKGROUND OF THE INVENTION

**THE PRESENT INVENTION** is directed, generally, to a method of using a synthetic selenoamino acid as a nutriceutical and its synthesis, and, more particularly, to a method of using and forming synthetic L-SE-methylselenocysteine.

Selenium (Se) is an essential nutrient for humans. Selenium is an essential nutrient because it fulfils the physiological requirement for at least thirteen human enzymes and proteins. One such protein is glutathione peroxidase, which provides for selenium's antioxidant role in the reduction of hydrogen peroxide and organic hydroperoxides to water and alcohols, respectively. In addition, selenium is incorporated into proteins via an amino acid, L-selenocysteine. These nutritional requirements for selenium are normally met by the consumption of cereal grains, meats and seafoods. Therefore, it should not be surprising that the present recommended daily allowance of selenium in the United States (U.S. RDA) for both men and women is 55 µg/Se/day. However, not all forms of selenium are required. In contrast, some selenoamino acids, such as L-Se-methylselenocysteine, are not incorporated into proteins, so they are not essential to the diet.

The metabolism of selenium in both animals and humans has also been characterized. In general, all organic selenoamino acids found in the diet, and selenite and selenate found in some dietary supplements, are believed to be ultimately reduced to hydrogen selenide, H₂Se. From hydrogen selenide, selenium may be phosphorylated and incorporated into proteins as the selenoamino acid, L-selenocysteine, formed from the co-translational modification of seryl-t RNA. Altematively, under normal dietary selenium ingestion, and during periods of excessive levels of dietary selenium ingestion, hydrogen selenide, H₂Se, is rapidly methylated, initially to methylselenol, then to dimethylselenide and finally to trimethylselenonium ion, the major excretory form of selenium found in urine. Because H₂Se is considered a very toxic form of selenium, methylation is the most likely pathway for selenium detoxification for urinary excretion. Furthermore, under conditions of toxic levels of selenium ingestion, the methylation pathway becomes rate limiting and dimethylselenide is further eliminated by pulmonary excretion.

Experimental data has indicated that selenium, when taken at the proper levels, has therapeutic anticarcinogenic effects in both animals and humans. However, additional data suggests that selenium, as selenite or selenomethionine, becomes a toxin at levels above 400 µg/day for a human. This is the official upper limit for a 70 kg human as established by the U.S. government. Accordingly, various dietary forms of selenium, particularly selenite, selenate, and selenomethionine and their concentrations have been analyzed to determine the extent to which these supplements may be used for the prevention or treatment of cancer.

Lu et al., in "Effect of an aqueous extract of selenium-enriched garlic on *in vitro* markers and *in vivo* efficacy in cancer prevention", *Carcinogenesis*, vol. 17, no. 9, pp. 1903-1907 (1996), showed that selenium-garlic extracts and racemic mixtures of Se-methylselenocysteine have beneficial anticancer effects on pre-neoplastic and tumor cell lines, as measured by cell morphology, cell number, and DNA strand breaks. In addition, Lu et al. showed a decreased tumor incidence, in a rat mammary tumor model, of rats fed Se-garlic extract at a concentration of 3 ppm of Se (which is equivalent to 1800 µg/day for an average adult human) in the diet, as compared to rats fed a regular garlic extract and control rats.

In addition, Iₚ et al., "Chemical Form of Selenium, Critical Metabolites, and Cancer Prevention", *Research,* vol. 51, pp. 595-600 (1991), have shown that supplementing rats' diets with a racemic mixture of Se-methylselenocysteine, in concentrations of 1 and 2 ppm (which is equivalent to 600 to 1200 µg/day for an average adult human, and is considered toxic), decreases the appearance of palpable mammary tumors in rats, using the dimethylbenzanthracene (DMBA) chemically induced mammary cancer model.

Furthermore, dietary L-selenomethionine has been reported to have an inhibitory effect on the promotional stages of rat colon carcinogenisis. In this treatment, rats are provided with dietary supplementation of selenomethionine at concentrations of 1.0-2.0 ppm to increase their N1 acetyl spermidine levels. Results of these studies suggest that selenomethionine may lower intracellular polyamines by inducing spermidine/spermine acetyl transferase.

It has also been reported that selenium enriched foods, such as broccoli, onions, and garlic provide some degree of anticarcinogenic activity. For example, it is known that selenium-enriched garlic may be grown and incorporated into the diet of rats that may reduce the incidence of chemical induced mammary tumors. Selenium enrichment is typically achieved by applying selenium, as selenate, on the leaves of each plant or placed at the roots of each plant to be adsorbed therein.

Research regarding the effects of selenium in humans has shown similar results as that in animals. Selenomethionine consumed as a dietary supplement up to 200 µg/Se/day more than that found in foods, has been reported to reduce the incidence of lung, cholorectal and prostate cancer in humans. This human research demonstrates that supplements of dietary selenium, beyond that required nutritionally to saturate all selenium proteins and enzymes, prevented and/or reduced cancer. Thus, the experimental data on humans agrees with the earlier published animal data showing the reduction in cancer by seleniurm at supranutritional dietary levels.

There is a difference between therapeutic and nutriceutical amounts of a compound. Compounds administered for therapeutic purposes are given in high doses to achieve a therapeutic effect. However, these high therapeutic doses can become toxic over time so they must be given for short periods of time. In contrast, compounds administered for nutriceutical purposes are given in low doses. These low nutriceutical doses are not toxic so they can be given everyday. To illustrate this further, selenium may be administered at a dosage of 1-30 mg/day, or 1-30 ppm/day, as a therapeutic modality for the direct treatment of cancer or as an adjunctive in combination with conventional methods of cancer treatment.

A number of methods have been developed to efficiently and effectively introduce additional *selenium* into a diet. Examples include ingestion of high selenium-containing foods, including gains, fruits, and vegetables, or by supplementation via oral ingestion, injection, and the like. Due to its natural relative abundance, selenomethionine is typically used for supplementation. While selenomethionine, the major dietary form of selenium, is naturally only found in cereal gains and animal proteins, yeast grown on a selenium containing medium can incorporate selenium into large amounts of selenomethionine, which allows it to be used as a supplement in this form.

In addition to the above, the results of several metabolic studies have shown that the generation of H₂Se is not necessary for the carcinostatic activity of selenium, but rather it is the continuous generation of the monomethylated selenium specio, methylselonol, that is responsible for selenium's anticancer activity. In addition, it has been suggested that methylselenol, CH₃SeH, may oxidize thiol compounds, such as cysteine residues of proteins and/or enzymes in a catalytic role, thereby transforming the redox environment of cells. Finally, it has been suggested that selenium's anti-carcinogenic activity may be due to a reduction in the blood supply to tumors, caused by restriction of capillary vessel development.

It is known that selenium compounds have anticarcinogenic activity only when consumed in amounts above normal dietary selenium levels, approximately 600 to 1200 µg/day. It is also known that there are differing thresholds of carcinostatic activity of selenium compounds *in vitro* or *in vivo*, greatly depending upon the chemical form of selenium. Based on this knowledge, it has been suggested that the anticarcinogenic property of selenium compounds is likely due to the known toxicity of selenium compounds, as studied in animals and man. Differences in the toxicity of selenium compounds *in vitro* and *in vivo* can be shown to be attributable, in part, to selenium's ability to generate superoxide in the presence of reduced glutathione (GSH) *in vitro.* All selenium compounds that can be readily reduced by GSH, forming the selenoate anion, RSe⁻, are capable of generating chemiluminescence (CL), which is indicative of superoxide generation in an *in vitro* chemical assay, In contrast, selenium compounds that do not form the selenoate anion by GSH reduction *in vitro* are dietarily less toxie to animals, are less toxic to cells *in vitro* and do not produce superoxide *in vitro* in the chemiluminescent assay.

However, the debate continues as to which chemical form of selenium most effectively inhibits the development and growth of cancer cells, and whether some selenium compositions have greater impact than others on certain types of cancer,

Several potentially useful naturally occurring forms of selenium exist that may provide significant cancer preventative properties. However, information on their toxicity and the ability to synthesize them on a large scale limits the practicality of their use. As a result, new synthetic methods are needed that provide a cost effective method of producing forms of selenium as nutriceuticals for their potentially cancer preventative properties.

### BRIEF SUMMARY OF THE INVENTION

The present invention addresses the above-mentioned debate and needs by providing a superior, yet economical, article of manufacture having a nutriceutically effective amount of L-Se-metylselenocysteine that will provide anti-cancer properties and yet not affect normal cells.

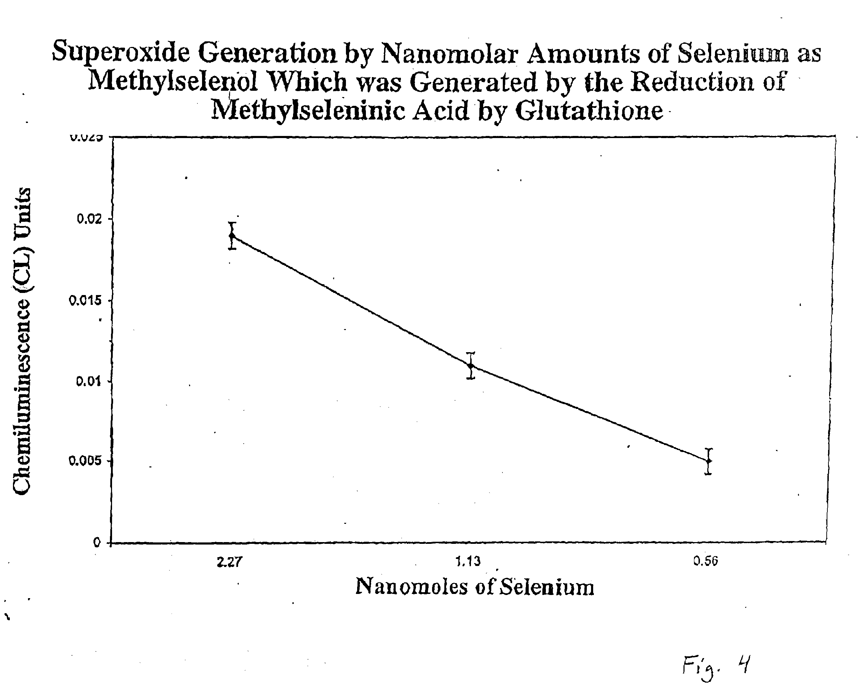

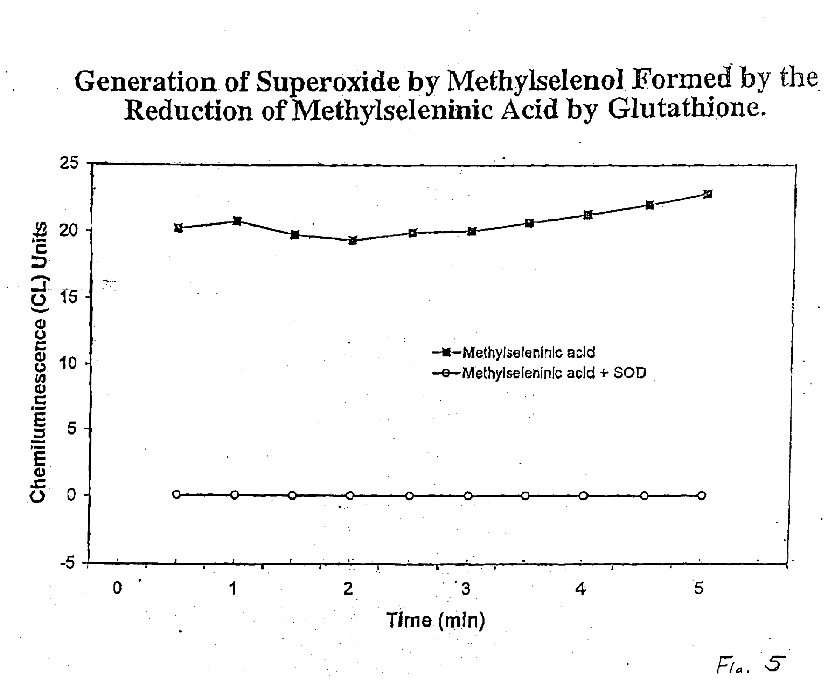

In addition, the present invention also provides a method of preventing or reducing the risk of developing cancer in mammals by administering a nutriceutically effective amount of synthetic L-Se-methylselenocysteine. Test data shows that L-Se-methylselenocysteine is slightly less toxic to normal cells than L-selenomethionine, which is unexpected bacause L-Se-methylselenocysteine is more toxic to cancer cells than L-selenomethionine. The present invention demonstrates that the mechanisms of both L-Se-methylselenocysteine (SeMC) and L-selenomethionine (SeMet) go through a common intermediate, methylselenol. Current data, as shown in Figures 4 and 5, show that methyl selenol, formed from methylseleninic acid, is highly catalytic for the production of superoxide radical and subsequently the production of hydrogen peroxide. Cancer cells preferentially use SeMC to produce methyl selenol, which results in the production of superoxide and hydrogen peroxide and leads to apoptosis (cell death).

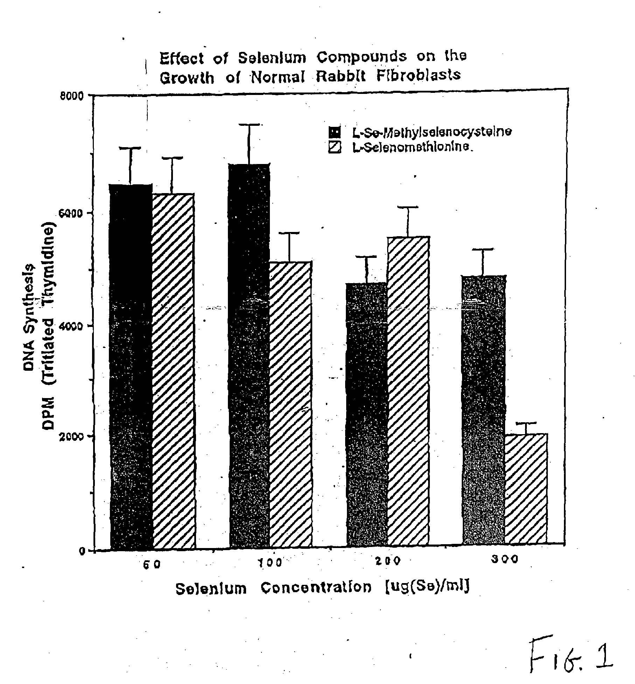

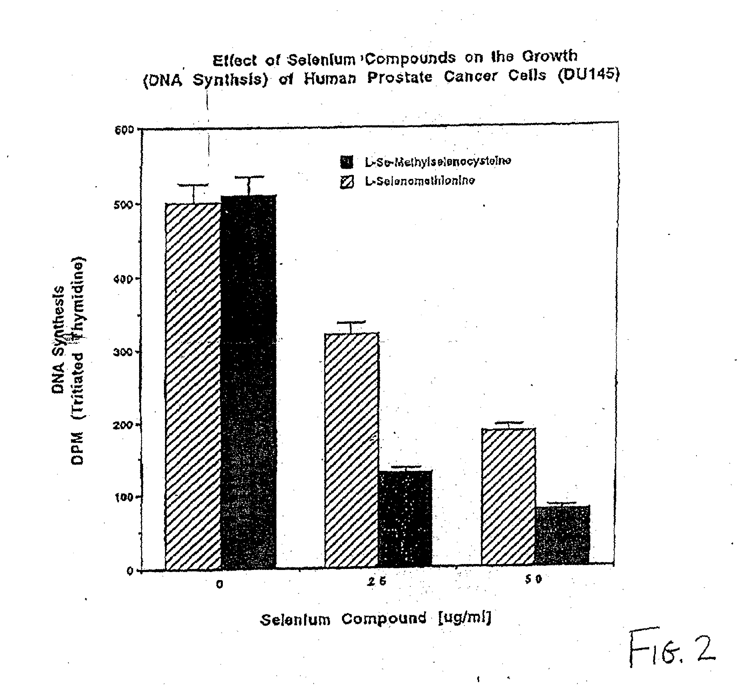

It is this mechanism which allows SeMC to be effective in causing cancer cells to die. There are two reasons why SeMC is better at causing apoptosis in cancer cells than SeMet. They are: 1) SeMC is a better substrate than SeMet for β-lyases in cancer cells, resulting in the formation of methylselenol; and 2) SeMC is not incorporated into proteins and thus, all of it is available for metabolism by cancer cells, as shown in Figure 2, where it is better for killing cancer cells than SeMet.

Therefore, our data teaches that SeMC should be more effective than SeMet in killing cancer cells. Thus, a dose of 300 µg/day or less is required to kill cancer cells in a normal human. This is less than the toxic dose of 600-1800 µg/day that is taught by Lu et al.

The present invention also provides a synthesis for forming the L-Se-methylselenocysteine.

The present invention provides an article of manufacture that includes from 7 to 300 micrograms of synthetic L-Se-methylselenocysteine and a non-toxic, pharmaceutically acceptable binder. The binder may be vitamins, minerals, herbals or starch. The binder may specifically be selected from calcium carbonate, magnesium hydroxide, magnesium sulfate, sodium tetraborate, cupric oxide, zinc sulfate, cholecalciferol, fumarate, pyridoxine hydrochloride, chromium picolinate, folate, or calcium phosphate and their salts.

The article of manufacture is used in a method for preventing or reducing the risk of developing cancer in mammals, preferably humans, but also dogs and cats. In accordance with the method, a nutriceutical amount of synthetic L-Se-methylselenocysteine is administered in the range of 7 to 300 micrograms/day.

In one form of the method of synthesizing L-Se-methylselenocysteine of the present invention, N-(tert-butoxycarbonyl)-L-serine is mixed with a dialkyl diazodicarboxylate and at least one of a trialkylphosphine, triarylphosphine, and phosphite to form a first mixture that includes N-(tert-butoxycarbonyl)-L-serine β-lactone. Methyl selenol or its salt is mixed with the N-(tert-butoxycarbonyl)-L-serine β-lactone to form a second mixture that includes N-(tert-butoxycarbonyl)-Se-methylselenocysteine. The tert-butoxycarbonyl group is removed from the N-(tert-butoxycarbonyl)-Se-methylselenocysteine to form L-Se-methylselenocysteme.

The selenium methylselenocysteine synthesis as herein described may be produced in quantities for use as a nutriceutical, rather than a therapeutic pharmaceutical, that provides various beneficial uses, such as, for example, preventing or reducing the risk of cancer. The present invention may also be used to synthesize selenium methylselenocysteine to be used alone or with various other minerals, vitamins, or nutrients as a broad based nutritional supplement to provide the prescribed recommended daily allowance (RDA) of micronutrients. The synthesized selenium methylselenocysteine may be used in combination with other materials, including, for example, pharmaceutical drugs and prescription medicines.

The synthesis of selenium methylselenocysteine as herein described, significantly improves the manufacturability, manufacturing efficiency, and utility of this form of selenium for supplementation into the diets of humans or animals.

### BRIEF DESCRIPTION OF THE SEVERAL VIEWS OF THE DRAWINGS

The characteristics and advantages of the present invention may be better understood by reference to the accompanying drawings, in which;
Figure 1 is a graphic comparison between L-Se-methylselenocysteine and L-selenomethionine and their effect on the growth of normal rabbit fibroblasts.
Figure 2 is a graphic comparison showing that L-Se-methylselenocysteine is more toxic to human cancer cells than L-selenomethionine.
Figure 3 is a graphic comparison showing the acute toxicity of L-Se-methylselenocysteine in a mouse versus the acute toxicity of selenomethionine in rats.
Figure 4 is a graphic illustration which demonstrates that methylselenol, generated by glutathione reduction of methylseleninic acid, can catalytically generate superoxide radicals.
Figure 5 is a graphic illustration which shows that superoxide dismutase can quench superoxide radicals that are generated by glutathione reduction of methylseleninic acid to methylselenol.

### DETAILED DESCRIPTION OF THE INVENTION

The present invention, as described herein, is particularly directed to an article of manufacture comprising a nutriceutically effective amount of synthetic L-Se-methylselenocysteine; a method of preventing or reducing the risk of developing cancer in mammals by administering a nutriceutically effective amount of synthetic L-Se-methylselenocysteine; and a method for synthesizing L-Se-methylselenocysteine. The method of the present invention may be used to synthesize L-Se-methylselenocystoine to be used alone or with various other minerals, vitamins, herbals, or nutrients. L-Se-methylselenocysteine may be used as a broad based nutritional supplement to provide the prescribed recommended daily allowance (RDA) of micronutrients. Also, the L-Se-methylselenocysteine, as synthesized and described herein, may be used in combination with other materials, including, for example, pharmaceutical drugs and prescription medicines. Thus, while the present invention is capable of embodiment in many different forms, for ease of description this detailed description discloses only specific forms as examples of the invention. Those having ordinary skill in the relevant art will be able to adapt the invention to application in other forms not specifically presented herein based upon the present description.

The present invention provides a synthesis of L-Se-methylselenocysteine using an economical synthetic routo to the compound, and knowledge that it is less toxic than L-selenomethionine to normal cells. Extracts of selenium enriched garlic have shown that L-Se-methylselenocysteine is the active ingredient in the chemoprevention of mammary cancer. L-Se-methylselenocysteine metabolism has been studied in animals and its chemopreveritive effect is believed to occur due to the generation of monomethylated selenium species by endogenous enzymes. Monomethylated selenium species have been shown to generate superoxide and to cause apoptosis to cancer cells in culture. Unlike L-selenomethionine that is incorporated into proteins in place of methionine, L-Se-methylselenocysteine is not incorporated into proteins and is, therefore, fully bioavailable for chemoprevention and the synthesis of selenium containing enzymes such as, for example, glutathione peroxidase. As L-Se-methylselenocysteine is only found naturally in some plants in the Allum family, including onions, garlic, leeks, and broccoli, and the Astragalas family, very little L-Se-methylselenocysteine is naturally incorporated into the human or animal diet. Accordingly, L-Se-methylselenocysteine is a very good candidate for human and animal dietary supplementation.

The synthesis of the present invention is an improvement of the synthesis of alpha-amino acids by Vederas et al, as discussed in Arnold, L, D., Kalantar, T. H., and Vederas, J. C. "Conversion of Serine to Stereochemically Pure β-Substituted α-Amino Acids via β-Lactones," *J*., *Am. Chem. Soc*., Vol, 107, pp. 7105-7109 (1985); Arnold, L. D., Drover, J. C. G., and Vederas, J. C. "Conversion of Serine β-Lactones to Chiral α-Amino Acids by Copper-Containing Organolithium and Organomagnesium Reagents," *J. Am. Chem. Soc.*, Vol. 109, pp. 4649-4659 (1987); Pansare, S. V., Huyer, G., Arnold, L. D, and Vederas, J. C. "Synthesis of N-Protected α-Amino Acids from N-(Benzyloxycarbonyl)-L-Serine via its β-Lactone: N°-(Benzyloxycarbonyl)- β-(pyrazol-1-yl)-L-alanine," *Org. Syn.*, Vol, 70, pp. 1-9 (1991); Pansare, S, V., Arnold, L. D, and Vederas, J.C. "Synthesis of N-(tert-Butoxycarbonol)-L-Serine β-Lactone and the p-Toluenesulfonic Acid Salt of (S)-3-Amino-2-oxetanone," *Org. Syn.*, Vol. 70, pp.-10-17 (1991); and Arnold, L. D., May, R. G. and Vedcras, J. C. "Synthesis of Optically Pure α-Amino Acids via Salts of α-Amino-β-Propiolactone," *J. Am. Chem, Soc.*, Vol. 110, pp. 2237-2241, (1998), which are incorporated herein by reference in their entirety. The synthesis of the present invention also incorporates some methodology reported by Sharpless, K. B. and Lauer, R. F. "A Mild Procedure for the Conversion of Epoxides to Allylic Alcohols, The First Organoselenium Reagent," *J. Am. Chem. Soc.*, Vol, 95, pp. 2697-2699 (1973); and Jones, J. *The Chemical Synthesis of Peptides*, pp. 22-23 (1991), which are incorporated herein by reference in their entirety.

The Vederas method teaches the production of amino acids based on the nucleophilic ring opening reaction of the β-lactone derived from serine. The β-lactone is subjected to deprotection, whereby the deprotected β-lactone is reacted with a nucleophile to form an alpha-amino acid. The Vederas method may be illustrated as follows:

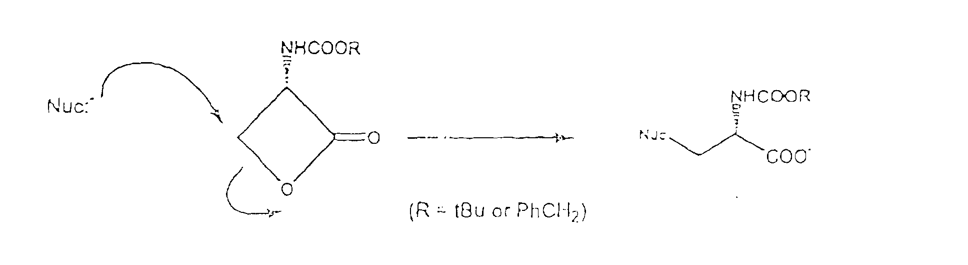

The improved synthesis of the present invention allows for the large scale production of L-Se-methylselenocysteine, and its incorporation into a nutritional supplement, which heretofore has been impractical. Generally, this synthesis includes treating N-protected serine derivatives, such as, for example, N-(tert-butoxycarbonyl)-L-serine with a dialkyl diazo-dicarboxylate, such as, for example, diethyl azodicarboxylate and a trialkylphosphine, triarylphosphine, or a phosphite such as, for example, triphenylphosphine to produce N-(tert-butoxycarbonyl)-L-serine β-lactone. The N-(tert-butoxycarbonyl)-L-serine β-lactone is added to methyl selenol, its salt, or combinations thereof to produce N-(tert-butoxycarbonyl)-Se-methylselenocysteine. The tert-butoxycarbonyl ("Boc") group may bo removed with an appropriate acid such as, for example, triflouroacetic acid, leaving the amine group, The overall reaction may be illustrated as follows:

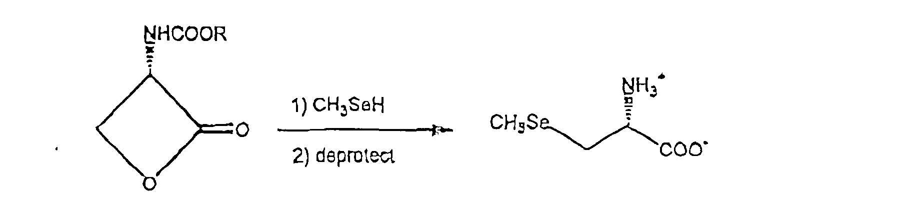

More specifically, and as more fully illustrated in Example 1, the synthesis of the present invention includes treating N-protected serine derivatives, such as, for example, N-Boc-L-serine with a dialkyl diazodicarboxylate such as, for example, diethyl azodicarboxylate (DEAD) or dimethyl diazodicarboxylate (DMAD) and a trialkylphosphine, triarylphosphine, or a phosphite such as, for example, triphenylphosphine to produce N-Boc-L-serine β-lactone A. The reaction may be illustrated as follows:

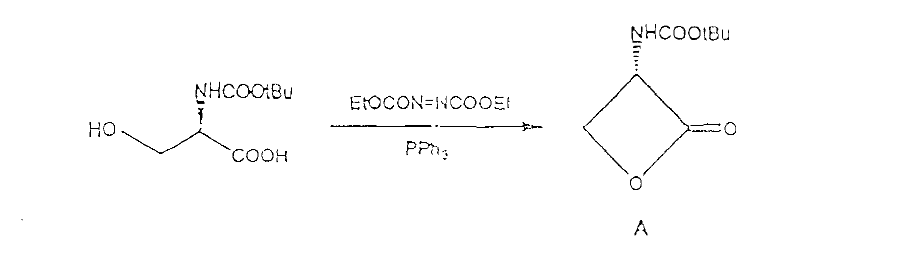

N-Boc-L-serine β-lactone **A** is a relatively stable solid, non-polar compound that can be handled on the benchtop, but because of its limited storage life, it should be used for further processing soon after it has been prepared. Methyl selenol, its salt, or combinations thereof, as the nucleophile may be produced by the reduction of dimethyldiselenide by sodium borohydride, in ethanol, or other reducing agents. A solution of N-Boc-L-serine β-lactone A in acetonitrile may bo added to the methyl selenol. The reaction mixture may be diluted with ethyl acetate, then extracted with a base, such as, for example, a 0.5 N NaOH solution. The combined aqueous extracts may be acidified with an acid, such as, for example, 20% HCl to a pH of about 1.0-5.0. The aqueous solution may be extracted with an organic solvent, such as, for example, ethyl acetate and dried, filtered, and concentrated to yield a light yellow oil. Column chromatography yields N-Boc-Se-methylselenocysteine **B**. The reaction may be illustrated as follows:

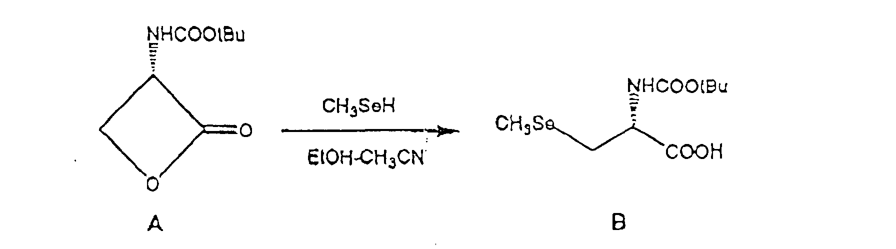

Treatment of the N-Boc-Se-methylselenocysteine with an acid (or Lewis acid), such as, for example, tifluoracetic acid (TFA) removes the Boc group and leaves only the amine group as a component. Treatment of N-Boc-Se-methylselenocysteine with TFA may be in dichloromethane to give the free amino acid in quantitative yield. The product obtained consists primarily of L-Se-methylselenocysteine **C**. The reaction may be illustrated as follows:

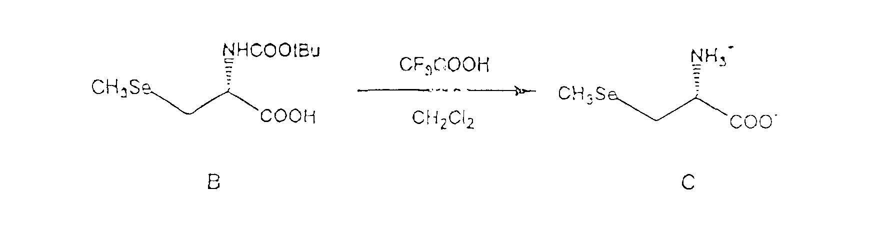

Example 1 illustrates a three-part synthesis of L-Se-methylselenocysteine: the preparation of N-(Boc)-L-serine β-lactone **A** (part A); the preparation of N-(Boc)-L-Se-methylselenocysteine **B** (part B); and the preparation of L-Se-methylselenocysteine **C** (part C). The following example is illustrative of the synthesis of the present invention and is not meant to limit the scope of the appended claims,

### EXAMPLES

### Example 1

### A- Preparation of N-(Boc)-L-serine β-lactone A

Triphenylphosphinc (3.166 g, 12.1 mmole, freshly opened and dried under vacuum over phosphorus pentoxide) was stirred in 50 mL dry tetrahydrofuran (THF) under argon at -78°C, and 1.9 mL (12.1 mmole) of diethyl azodicarboxylato (freshly opened, but not distilled), in the form of en orange oil, was added drop wise, followed by THF rinses of the syringe. To the resulting clear yellow solution, a solution of 2.4 g (11.7 mmole) of N-Boc-L-serine (freshly opened and dried under vacuum over phosphorus pentoxide), in 50 mL dry THF, was added drop wise, followed by THF rinses of the addition funnel. The resulting pale yellow suspension of solid was stirred at -78°C for 15 minutes, followed by removal of the cooling bath. The mixture, which turned clear and colorless as it warmed, was allowed to warm to room temperature and to stir for 1.5 hours, The mixture was concentrated by rotary evaporation and triturated with 85:15 hexanes:ethyl acetate. The triturant was chromalographed on 21 g 230-400 mesh silica gel, using a gradient elution of 85:15 hexanes:ethyl acetate. The product **A** quickly came out in the first fractions. Furthermore, thin layer chromatography ("TLC") analysis showed that the product was still present in the solid left behind by the trituration procedure; chromatography of it yielded more of the product **A**. Altogether, 2.5 grams of a semi-solid consisting of the β-lactone **A** contaminated with triphenyl phosphine and triphenyl phosphine oxide was obtained (theoretical yield of pure β-lactone was 2.2 g), which was carried on into the next synthesis phase without further purification.

TLC for this synthesis included use of silica gel plates, 80:20 hexanes:ethyl acetate as the developing solvent, and I₂ visualization. The product had an R_{f} of approximately 0.7 in this system.

It is contemplated that during small scale operations, as described above, the trituration following the addition of N-Boc-L-serine may not be necessary. Trituration may only be necessary when the reaction is scaled up, to remove most of the triphenyl phosphine oxide (TPPO). If trituration is not used, the reaction mixture may be concentrated by rotary evaporation, mixed with enough toluene to dissolve the TPPO, and the solution then applied to the top of a silica gel column packed in 90;10 hexanes:ethyl acetate, and eluted with 90:10 hoxanes:ethyl acetate. The product may be observed as a TLC spot which is only slightly more polar than triphenyl phosphine, and which can be best visualized by I₂ vapors. If the TPPO precipitates on the column, then the mixture may be eluted with dichloromethane.

### B. Preparation of N-(Boc)-L-Se-methylselenocysteine B

Dimethyl diselenide (0.76 mL (8 mmole), freshly opened) was stirred in 30 mL absolute ethanol at 0°C under argon. Sodium borohydride (NaBH₄) in an amount of about 0.5 grams (13 mmole) was added as the solid, in portions, over a 10-minute period. Vigorous bubbling occurred upon each addition, and the yellow color of the diselenide disappeared as the reaction proceeded. The solution was stirred at 0°C for 10 minutes following the final NaBH₄ addition, then a solution of the β-lactone **A**, in 15 mL acetonitrile, was added drop wise, resulting in a cloudy, colorless solution. This solution was stirred at 0°C under argon for 2 hours, and 100 mL ethyl acetate was added, whereupon the solution was transferred to a separatory funnel. The solution was extracted with 0.5 N NaOH solution (4 x 25 mL). The combined aqueous extracts were acidified with 20% HCl solution to about pH 2.0. The mixture was extracted with fresh ethyl acetate (3 x 30 mL), The combined organic extracts were dried (MgSO₄), filtered, and concentrated by rotary evaporation (under high vacuum) to yield a light yellow oil weighing about 0.85 grams, which amounted to approximately 26% yield from the N-Boc-L-serine starting material. This material was mixed into dichloromethane and deposited atop a column of 50 g 230-400 mesh silica gel, pre-equilibrated with 80:20:1 hexanes:ethyl acetate:acetic acid, and eluted with 400 mL of 80:20:1 hexanes:ethyl acetate:acetic acid, then with 400 mL of 50:50:1 hexane:ethyl acetate:acetic acid, and then with 400 mL of 50:50:1 hexanes:ethyl acetate:acetic acid to yield impure product **B**. Rechromatography on 21 g 230-400 mesh silica gel, using 85:15:1 (150 mL), then 80:20:1 (200 mL), then 75:25:1 (200 mL) hexanes:ethyl acetate:acetic acid in sequence, yielded 0.19 g of pure N-Boc-Se-methylselenocysteine **B**, as a waxy solid.

This reaction can be repeated with TLC monitoring to observe the appearance of the product as well as the disappearance of the starting material.

TLC for this synthesis included using silica gel plates and a developing solvent consisting of 50:50 hexanes:ethyl acetate to which several drops of acetic acid had been added, I₂ was used as the visualization agent. Under these conditions, the product had an R_{f} value of about 0.5.

It is contemplated that additional time and additional amounts of NaBH₄ could be provided that may allow for the complete reduction of the dimethyl diselenide. Also, it is possible that use of a larger excess of methyl selenol may be beneficial to allow for a more complete reaction to occur, as unreacted β-lactone may be present in the reaction mixture that may be subsequently lost during the acid-base extraction.

It is also contemplated that the 20% HCl acidification used during the acid-base workup may have hydrolyzed the Boc group from the desired N-(Boc)-L-Se-methylselenocysteine product, producing the free amino acid which was subsequently lost during chromatography. Also, a saturated citric acid for acidification of solutions containing N-Boc compounds may be used. Accordingly, it is contemplated that saturated citric acid could be used in place of the 20% HCl for the acid-base extraction method described previously.

The acid-base extraction may be used to remove (in the initial organic wash) as much of the volatile and highly odoriferous methyl selenol (or unreacted dimethyl diselenide) as possible. An alternative procedure could be to perform a standard aqueous/organic workup (add water to the reaction mixture and extract with ethyl acetate, then dry, filter and concentrate) and renove the odoriferous compounds by vacuum treatment prior to chromatography. This alternative approach might improve the yield of the reaction, and reduce the losses incurred during the base extraction and acidification steps. Once the pure N-Boc-Se-methylselenocysteine is obtained, the odor produced is relatively minor.

### C. Preparation of L-Se-methylselenocysteine C

A solution of 0.156 g (0.55 mmole) of N-(Boc)-L-Se-methylselenocysteine **B**, in 5 mL dichloromethane under argon at room temperature, was mixed with about 1 mL of freshly opened trifluoroacetic acid (TFA). The solution was stirred at room temperature for 25 minutes. It was then concentrated by rotary evaporation, and dry ether (about 30 mL) was added, then removed by rotary evaporation. The ether treatment was repeated twice, resulting in the formation of a fine white solid after solvent removal. Treatment under high vacuum yielded L-Se-methylselenocysteine **C**, as an off-white solid, 0,195 g (>100%). This material was mixed with approximately 2 mL of boiling water, and the solution was filtered through a plug of glass wool. Upon cooling, the water was azeotroped off using copious ethanol, and high vacuum treatment provided 0.16 g of L-Se-methylsolenocysteine **C**. Homogeneity of the amino acid was determined by TLC using "Chiralplates" (i.e., reverse phase TLC plates which also are useful for assessing optical purity) provided by Aldrich Chemical Company, Milwaukee, Wisconsin, (Cat, No, Z14870-9) using a solvent system of 4:1:1 acetonitrilemethanol:water which resulted in a single ninhydrin-positive spot with an R_{f} value of about 0.4.

TLC for this synthesis included use of Chiralplates which were found to be useful for visualizing the free amino acid. A solvent system of 4:1:1 acetonitile:methanol:water was found to produce a ninhydrin-positive spot with an R_{f} value of about 0.4. A ninhydrin "dip" reageint (approximately 0.1% ninhydrin in ethanol) was used to visualize the product.

It is contemplated that ion exchange chromatography may be used to purify the amino acid. It is also possible that, on a larger scale, L-Se-methylselenocysteine may be crystallized. In this regard, the L-Se-methylselenocysteine obtained was fairly soluble in methanol, soluble in water, and sparingly soluble in ethanol. Accordingly, ethanol may be one likely crystallization solvent.

### Example 2

It is contemplated that the synthesis described in Example 1 may be simplified. Generally, serine β-lactone lacking the N-Boc group can be produced with the "free" amino group (i.e., without the N-Boc group), as a salt, and that this β-lactone will react with nucleophiles to produce, upon workup, the free amino acids directly from the β-lactone. The β-lactone, lacking the N-Boc group, can be generated from the N-Boc β-lactone for use *in situ*, or it can be isolated as a tosylate salt. Accordingly, it is believed that methyl selenol can react with the "unprotected" β-lactone, to produce L-Se-methyl selenocysteine via a more simplified synthesis. This reaction may be illustrated as:

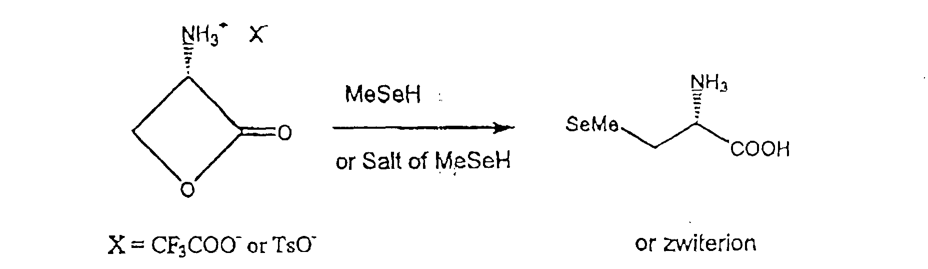

The Boc group was retained during the previously illustrated synthesis to obtain easily isolable and identifiable non-water soluble, "organic" intermediates to confirm that the chemistry was proceeding as desired. Because it has been shown that the β-lactone can be formulated in this manner, and that it undergoes ring opening with methyl selenol, the more simplified approach can be performed.

### Example 3

Example 3 illustrates the method of preventing or reducing the risk of developing cancer in mammals by administering an effective nutriceutical amount of synthetic L-Se-methylselenocysteine.

### Materials and Methods

Methylseleninic acid (CH₃SeOOH) was a dissolved in distilled water. Using a Rannin micropipette, aliquots were added directly to 1.0 ml of a buffered cocktail solution. The buffered cocktail solution was comprised of lucigenin (20 µg/ml), reduced glutathione (GSH) (4 mg/ml), and either sodium borate (0.05M) or sodium phosphate (0.05M), all purchased from Sigma Chemical Co. Two different buffered cocktail solutions were employed: the cocktail containing sodium borate was used for experiments at pH 9.2, which is optimum for the generation, detection and quenching of selenium catalyzed superoxide; and the cocktail containing sodium phosphate was used for experiments at pH 7.4, which is reflective of physiological conditions. One ml of the cocktail solution was added to a 10 X 50 mm polypropylene tube for use in the chemiluminescence (CL) assay. Chemiluminescence, using lucigenin as the detector of superoxide, was counted in repetitive integrated 30 second increments over time using a Los Alamos Diagnostics (Model 535) chemiluminometer, to which was attached a LKB Model 2209 circulating water bath that held the tube at 36°C. All chemical test assays and controls were performed by the addition of methylseleninic acid directly to the luminometer test tube in the counting chamber. Superoxide dismutase, SOD, purchased from Sigma Chemical Co., was added to the test tube prior to the addition of the methylseleninic acid and used to quench the chemiluminescent reactions.

### Results

The complete chemiluminescence cocktail (containing buffer, lucigenin and GSH) produced very low, yet detectable amounts of background chemiluminescence at both a pH of 9.2 and 7.4. This was most likely due to the ambient spontaneous oxidation of GSH, but was not significant to the tests performed. At both pH concentrations, 9.2 and 7.4, methylseleninic acid, in the presence of lucigenin alone, i.e., without GSH, produced no additional chemiluminescence. Similarly, methylseleninic acid, in the presence of GSH alone, i.e., without lucigenin, produced no additional chemiluminescence. Thus the chemiluminescence produced and quantitated in these assays can be attributable to the generation of superoxide. The native superoxide dismutase quenches the chemiluminescence generated in these assays, whereas heated and denatured superoxide dismutase does not quench the chemiluminescence. The chemiluminescence generated by selenoate catalysis may be used to quantitatively approximate the amount of catalytic selenium present.

Methylseleninic acid generated superoxide at pH 9.2, the optimum catalytic pH for the SOD enzyme. The addition of 100 units of SOD prior to the addition of methylseleninic acid quenched an initial burst of chemiluminescence activity that was seen. Methylseleninic acid also produced chemiluminescence at physiological pH 7.4. However, at pH 7.4, no burst of chemiluminescence was generated, and less overall chemiluminescence activity was generated than at the higher pH. In addition, 100 units of SOD quenched most of the chemiluminescence activity. As shown in Figure 4, chemiluminescence (superoxide generation) from the addition of methylseleninic acid could be detected down to a level of 0.56 nanomoles of selenium.

Selenium compounds are known to be toxic to cells both *in vitro* and *in vivo*, with absolute toxicity depending upon the chemical form of selenium, its concentration and its metabolism. In general, selenite and diselenides are very toxic to cells in culture and to animals. On the other hand, L-selenomethionine and L-Se-methylselenocysteine arc not very toxic to cells in culture, nor arc these selenium compounds very toxic to animals *in vivo*, relative to selenite or diselenides. For example, selenite induces DNA ladders and apoptosis in cells, whereas much higher concentrations, approximately 8-10 fold, of L-selenomethionine and L-Se-methylselenocysteine are required to induce apoptosis and cell death.

*In vitro,* selenite and diselenides examined and tested under the experimental conditions described herein, generate superoxide (O₂), as measured by chemiluminescence. In addition, SOD is able to quench the chemiluminescence produced by methylseleninic acid. Glutathione's ability to reduce diselenides, as well as other thiols, to form selenoate anion (RSe⁻), has been described in detail, However, methylselenol from methylseleninic acid has not heretofore been shown to produce catalytically, superoxide. Selenoate anion from methylselenol has now been shown to redox cycle indefinitely in the presence of GSH and oxygen, continuously generating superoxide and hydrogen peroxide (H₂O₂). It is this redox cycling of selenoate anion that appears to account for selenium's toxicity both *in vitro* as well as *in vivo* and for the anti-carcinogenic activity of Se-methylselenocysteine.

Unlike the methylseleninic acid, the monoselenide dietary amino acids, L-selenomethionine (CH₃SeCH₂CH₂CHNH₂COOH) and L-Se-methylselenocysteine (CH₃SeCH₂CHNH₂COOH), are not reducible to selenide anion (RSe⁻) by glutathione or other thiols, such as dithiothreitol, *in vitro* and therefore do not redox cycle *in vitro.* These selenoethers do not generate superoxide in the *in vitro* chemiluminescence assay, and, as noted above, arc found to be much less toxic to cells *in vitro* and to animals *in vivo.* However, L-selenomethionine and L-Se-methylselenocysteine are known to be toxic to cells *in vitro* at much higher selenium concentrations in comparison to selenite, and *in vivo* to animals at much higher than normal selenium dietary levels. In addition, it has been shown that the monomethyl specie of selenium, CH₃SeH, must be continuously generated from selenium compounds in order to have carcinostatic activity.

It was believed that the monomcthylated selenium specie, methylselenol, CH₃SeH, formed upon the reduction of methylseleninic acid by GSH, should be a highly reactive redox cycling species in its ionized form because the pKa of selenol in selenocysteine is 5.25, As described in the experimental section above, methylselenol, derived from the reduction of methylseleninic acid, is a very active redox compound producing superoxide. This experience also suggests that this selenonium specie, methylselenol, is the most catalytically active of any diselenides reduced by GSH that we have tested.

In contrast to methylselenol, the corresponding sulfur specie, methylthiol, CH₃S, was found to generate only a fraction of the amount of chemiluminescence as compared to methylselenol. The pKa of the thiol of cysteine is approximately 8-9, The very small amount of chemiluminescence generated by methylthiol appeared not to be due to superoxide because SOD did not significantly quench the chemiluminescence. The chemiluminescence generated by methylthiol, although not due to superoxide, may be due to the transient generation of some other free radical species upon reduction with glutathione, such as a thiol radical or glutathione radical. The inability of CH₃S⁻, as well as other thiols, to redox cycle may explain why these sulfur compounds arc not nearly as toxic as selenols or as effective as selenols in the prevention of cancer.

Several experiments provide a plausible understanding of how the dietary selenium compounds, L-selenomethionine and L-Se-methylselenocysteine, arc metabolized to produce an RSe⁻ selenium specie, which can redox cycle to induce apoptosis and cancer cell death by the initial generation of superoxide free radical. *In vivo*, it is metabolically possible to form two redox selenium species of L-selenomethionine and L-Se-methylselenocysteine. The amino acid RSe⁻ could be formed by a demethylation reaction, which would be fully expected to redox cycle, generating superoxide. In fact, demethylation of methylated selenium compounds is known to occur *in vivo*. Monomethylated and dimethylated selenium compounds and even the trimethylselenonium ion can function dietarily, if in sufficient concentration, to support the synthesis of the selenoenzyme, glutathione peroxidase. However, the synthesis of glutathione peroxidase could happen only if selenium demethylation reactions could take place to form H₂Se, as has been demonstrated for the trimethylselenonim ion. Therefore, the selenonium anion of these selenoamino acids is not likely to be formed. More likely to be formed is methylselenol.

Highly active β-lyases are reported to be prevalent in renal carcinoma cells, where β-lyase activity and carcinostatic activity was inhibited by the addition of the β-lyase inhibitor, aminooxyacetic acid. β-lyases may also be present in other cancer cells, forming the basis for the generation of methylselenol from L-selenomethionine and L-Se-methylselenocysteine *de novo.* Therefore, it seems highly likely that dietary selenoether species (CH₃SeR) such as L-selenomethionine and L-Se-methylselenocysteine need only be delivered to cancer cells continuously, at sufficient concentrations, to generate the highly catalytic methylselenol in the presence of β-lyases to produce superoxide, thus inducing oxidative stress, apoptosis, and over time, having carcinostatic activity.

The data supporting the present invention shows that L-Se-methylselenocysteine is more effective as a chemopreventative selenium nutriceutical than L-selenomethionine. Several factors likely account for these observations. The first is that selenomethionine is incorporated into proteins in place of methionine, effectively reducing its availability to generate methylselenol. Second, L-Se-methylselenocysteine is a non-protein accumulating selenoamino acid so it is freely available to metabolically provide methylselenol, In addition, these two selenoamino acids act differently as substrates for β-lyase cleavage. Additionally, there may be an unknown pathway to generating methylselenol. It has been suggested that L-Se-methylselenocysteine may follow the metabolic pathway of S-methyleysteine, whose hydrolysis would yield serine and methylselenol. Thus there are likely several metabolic possibilities for the generation of methylselenols from these selenoamino acids.

Experiments were conducted to demonstrate the association of superoxide generation and the toxicity of methylselenol with cells *in vitro*. It was determined that superoxide can be detected *in vitro* from the catalytic activity of methylselenol at selenium levels found to be toxic to cells ex *vivo*, Measurements were made of superoxide generation by methylseleninic acid in the presence of GSH at levels that induced apoptosis in cells in culture. Thus, it appears that methylselenol can generate detectable amounts of superoxide at levels that may induce oxidative stress in cells, producing apoptosis and cell death.

One additional line of evidence suggests that this is true. A selenium molecule, having the configuration RSe⁻, was covalently attached to a variety of antibodies, peptides, a steroid, and polymeric surfaces. All were shown to generate superoxide *in vitro.* In addition, these site directed or surface selenols are only toxic to cells if bound to them in very close proximity or if they are phagocytized. This suggests that there exists a close, if not identical, cause of cell death by chemoprevention using selenoamino acids as dietary agents or using selenium bound vector molecules to treat disease.

L-Se-methylselenocysteine, as synthesized above, may be produced in large quantities for incorporation into nutriceuticals for various beneficial uses, such as, for example, to prevent or reduce the risk of developing cancer in humans and animals, including household pets, Relative to L-selenomethionine, L-Se-methylselenocysteine is not incorporated into proteins (resulting in L-Se-methylselenocysteine being considerably less toxic than expected - see Figure 1) and, by the synthesis of the present invention, is easier to include in nutriceuticals. Accordingly, L-Se-methylselenocysteine may be a more effective cancer preventative nutriceutical than L-selenomethionine at smaller doses.

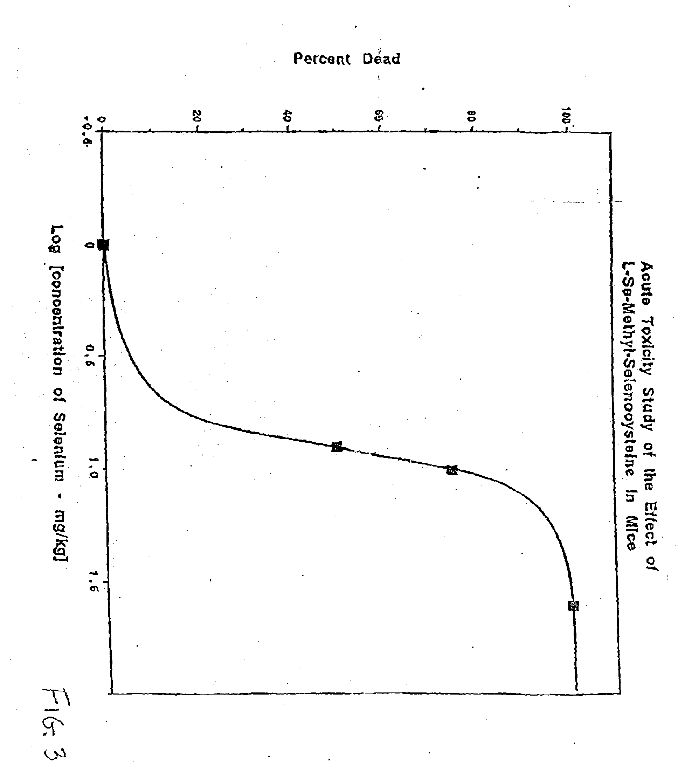

Figure 1 illustrates a comparison between L-Se-methylselenocysteine and L-selenomethionine, and their effect on the growth of normal rabbit fibroblasts. Tests were performed at varying concentrations of L-Se-methyselenocystein and L-selenomethionine over a three-day period. The amount of cellular growth, as measured by DNA synthesis, that could be stimulated by calf serum at the end of this period was assessed by the addition of tritiated thymidine. As illustrated, test results unexpectedly indicate that L-Se-methylselenocysteine is less toxic than L-selenomethionine, based upon the previous experimental results that showed that L-Se-methylselenocysteine is more toxic than L-selenomethionine to cancer cells (see Figure 2). Figure 2 shows that L-Se-methylselenocysteine is more toxic than L-selenomethionine when added to cancer colls growing according to the same protocol as that used for the treatment of normal cells. As illustrated in Figure 3, the acute toxicity of L-Se-methylselenocysteine in a mouse yields an LD₅₀ of 8.0 mg/kg, which is better than the LD₅₀ of 4.25 mg/kg reported for selenomethionine in rats (Spallholz, 1994). Thus, L-Se-methylselenocysteine is less toxic to normal cells and more toxic to cancer cells than selenomethionine, while at the same time showing a similar LD₅₀ value for acute toxicity.

It has been shown that L-Se-methylselenocysteine and selenomethionine work by the same mechanism in killing cancer cells, Both of these compounds generate methyl selenol in cells *in vitro* and *in vivo.* Experiments show for the first time that methyl selenol alone can generate superoxide radicals, which have been shown to induce apoptosis in cancer cells, Because L-Se-methylselenocysteine and selenomethionine utilize the same mechanism for cellular killing, and because selenomethionine has been shown in clinical trials to inhibit cancer formation in humans at a concentration of 200 µg/day, L-Se-methylselenocysteine may be utilized as a nutriceutical at concentrations of 200-300 µg/day for an adult human. This is equivalent to 0.2-0.3 ppm of dietary selenium, which is much less than the therapeutic levels shown by others. In addition, because L-Se-methylselenocysteine is not incorporated into proteins, as is selenomethionine, it is likely that L-Se-methylsolenocysteine will function as an effective nutriceutical at even lower doses, such as 7-200 µg/day, or 100-200 µg/day, 7-100 µg/day, or even 50-100 µg/day.

L-Se-methylselenocysteine may be used in a stand-alone form for supplementation for humans or animals, including cats or dogs, Based upon the sbove results, if used for human supplementation, L-Se-methylselenocysteine may be used, for example, in tablet form at levels of 7-300 µg/day of selenoamino acid. To extend this to a weight average, assuming the pharmacological standard of a 70 kg person, then one would use 0.1-4.3 µg/kg of selenoamino acid as a nutriceutic modality for the prevention of cancer or as an adjunctive in combination with conventional methods of cancer prevention. If used for animal supplementation, L-Se-methylselenocysteine may be used, for example, in tablet form at levels of 1-2 µg Se/kg/day of selenoamino acid.

L-Se-methylselenocysteine may be combined with vitamins, minerals, herbals, and other compounds to form supplements that are specifically included to address common health concerns. These supplements may be formulated using any pharmaceutically acceptable forms of the vitamins, minerals, herbals, and other nutrients, including their salts, such as, for example, calcium carbonate, magnesium hydroxide or magnesium sulfate, sodium tetraborate, cupric oxide, magnesium sulfate, zinc sulfate, cholecalciferol, fumarate, pyridoxine hydrochloride, chlorine picolinate, Vitamin B₆, folate, and other well known dietary components.

Although the supplements formed with L-Se-methylselenocysteine may be used in combination with other vitamins, minerals, herbals, or nutrients in a broad based nutritional supplement to provide the prescribed recommended daily allowance (RDA) of micronutrients, the present invention is geared to emphasize the disease prevention properties of micronutrient supplementation, and their cumulative beneficial and preventative effects related to the prevention of cancer. In this regard, the L-Se-methylselenocysteine of the present invention may be combined with any compatible nutrient that functions as an antioxidant, such as, for example, Vitamin E and Vitamin C. When combined with natural or man-made Vitamin E, the synthesized L-selenium methylselenocysteine may range from 7-300 µg/day. Moreover, the synthesized L-selenium methylselenocysteine may be combined with L-selenomethionine, selenite, sclenate, selenium yeast (i.e., yeast grown in the presence of selenium) or combinations thereof, or other selenium components. The supplements may also contain "fillers" such as starch or calcium phosphate.

The supplements formed with L-Se-methylselenocysteine may be formulated into any form, such as, for example, capsules, tablets, powders, gels, or liquids. Also, the supplements may be mixed with consumable liquids such as, for example, milk, juice, water, gels, or syrups. Moreover, these dietary supplements may be formulated with other foods or liquids to provide pre-measured supplemental foods such as, for example, a single serving bar. Flavorings, binders, protein, complex carbohydrates, and the like may also be added.

It is contemplated that the nutriceuticals with L-Se-methylselenocysteine may be administered daily, or may be formulated in multiple portions for more frequent administration, or as time release compositions for less frequent administration. For example, the nutriceutical may be formulated as two tablets for twice daily administration or as a sustained release capsule for relatively even administration over two days. For reasons of size (ease of swallowing) or improved bioabsorption or utilization (e.g., before or after a meal or before sleep), a given dosage may be divided into two, three, or more tablcts (or capsules, etc.). A daily dosage may be administered as one tablet, as two tablets taken together, or as two tablets taken separately (e.g., one in the morning and:one in the evening).

The preferred method of delivery of the described invention is by oral ingestion. However, any other suitable delivery system may be employed, such as, for example, enternal to the stomach or digestive track, injection, or I.V parenteral solution form as determined by medical and/or nutritional professionals. For example, in cancer patients, an intubation (stomach tube) or parenteral delivery system may be employed.

Although the foregoing description has necessarily presented a limited number of embodiments of the invention, those of ordinary skill in the relevant art will appreciate that various changes in the components, materials, or synthesis arrangement as have been herein described and illustrated in order to explain the nature of the invention may be made by those skilled in the art, and all such modifications will remain within the principle and scope of the invention as expressed herein in the appended claims. In addition, although the foregoing detailed description has been directed to an embodiment of L-Se-methylselenocysteine in the form of a nutritional supplement to prevent or reduce the risk of cancer in humans or animals, it will be understood that the present invention has broader applicability and, for example, may be used in combination with other vitamins, minerals, herbals, or nutrients, or with pharmaceutical drugs or medicine to provide additional beneficial properties. All such additional applications of the invention remain within the principle and scope of the invention as embodied in the appended claims.

It is to be appreciated that the method may be considered to comprise a method of preventing or reducing the risk of developing cancer in mammals comprising administering a nutriceutical amount of synthetic L-SE-Methylselenocysteine in the range of 7-300 micrograms daily. The mammals may be human, in which case the amount is in the range of 7-200 micrograms a day, such as 7-100 micrograms a day, or preferably 50-200 micrograms a day. Alternatively the amount is in the range of 200-300 micrograms a day. Alternatively the animals are selected from the group consisting of cats and dogs and the amount is in the range of 1-5 micrograms of selenium/kg of bodyweight/day, or preferably in the range of 1-2 micrograms of selenium/kg of bodyweight/day.

In the present Specification "comprise" means "includes or consists of" and "comprising" means "including or consisting of".

The features disclosed in the foregoing description, or the following Claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. An article of manufacture 7 to 300 micrograms of synthetic L-Se-methylselenocysteine, and a non-toxic, pharmaceutically acceptable binder.

2. The article of Claim 1 wherein said binder is selected from the group consisting of vitamins, minerals, herbals and starch.

3. The article of Claim 1 or 2 wherein said binder is selected from the group consisting of calcium carbonate, magnesium hydroxide, magnesium sulfate, sodium tetraborate, cupric oxide, zinc sulfate, cholecalciferol, fumarate, pyridoxine hydrochloride, chlorine picolinate, folate, calcium phosphate and their salts.

4. A method of producing Le-Se-methylselenocysteine, comprising mixing N-(tert-butoxycarbonyl)-L-serine with a dialkyl diazodicarboxylate and at least one of a trialkylphosphine, triarylphosphine, and phosphite to form a first mixture that includes N-(tert-butoxycarbonyl)-L-serine β-lactone, mixing at least one of methyl selenol and a salt of methyl selenol with the N-(tert-butoxycarbonyl)-L-serine β-lactone to form a second mixture that includes N-(tert-butoxycarbonyl)-Se-methylselenocysteine, the N-(tert-butoxycarbonyl)-Se-methylselenocysteine having a tert-butoxycarbonyl group, and removing at least one tert-butoxycarbonyl group from the N-(tert-butoxycarbonyl)-Se-methyiselenocysteine to form the L-Se-methyiselenocysteine.

5. The method of Claim 4 wherein said mixing to form a first mixture further includes mixing tetrahydrofuran with the first mixture under argon.

6. The method of Claim 4 OR 5 wherein the dialkyl diazodicarboxylate is diethyl azodicarboxylate, and the at least one of trialkylphosphine, triarylphosphine, and phosphite is triphenylphosphine.

7. The method of any one of Claims 4 to 6 wherein said removing comprises mixing trifluoroacetic acid with the second mixture.

8. The method of any one of Claims 4 to 7 wherein said mixing to form a first mixture occurs for about 15 minutes under a cooling bath at approximately -78°C.

9. The method of Claim 8 further comprising allowing said first mixture to warm to room temperature following said mixing to form the first mixture.

10. The method of Claim 9 further comprising concentrating the first mixture by rotary evaporation following said mixing to form the first mixture.

11. The method of Claim 10 further comprising titrating the first mixture with 85:15 hexanes:ethyl acetate following said mixing to form the first mixture.

12. The method Claim 10 further comprising chromographing the first mixture using a gradient elution of 85:15 haxanes:ethyl acetate following said mixing to form the first mixture.

13. The method of any one of Claims 4 to 12 wherein sodium borohydride is mixed with the at least one of methyl selenol and salt of methyl selenol prior to obtaining the first mixture.

14. The method of any one of Claims 4 to 13 wherein the second mixture is mixed under argon at 0°C.

15. The method of any one of Claims 4 to 14 further comprising acidifying the second mixture with hydrochloric acid to a pH of about 2.0.

16. The method of any one of Claims 4 to 15 wherein said removing comprises mixing trifluoroacetic acid with the second mixture.

17. The method of Claim 16 wherein said removing further comprises mixing the second mixture in dichloromethane under argon at room temperature.

18. A method of producing L-Se-methylselenocysteine, comprising mixing at least one of methyl selenol and a salt of methyl selenol with the L-Se-methylselenocysteine.

19. A method of Claim 18 further comprising making L-serine β-lactone by mixing L-serine with a dialkyl diazodicarboxylate and at least one of a trialkylphosphine, triarylphosphine, and phosphite to form the L-serine β-lactone.

20. The method of Claim 19 wherein the dialkyl diazodicarboxylate id diethyl azodicarboxylate, and the at least one of trialkylphosphine, triarylphosphine and phosphite is triphenylphosphine.
